## Europäisches Patentamt

## European Patent Office

## Office européen des brevets

(19)

(11) Publication number: **0 030 094**
A1

(12)

# EUROPEAN PATENT APPLICATION

(21) Application number: **80304065.8**

(22) Date of filing: **13.11.80**

(51) Int. Cl.³: **C 12 P 21/00**
//C12R1/91

(30) Priority: **30.11.79 GB 7941377**

(43) Date of publication of application:
**10.06.81 Bulletin 81/23**

(84) Designated Contracting States:
**AT BE CH DE FR GB IT LI NL SE**

(71) Applicant: **BEECHAM GROUP LIMITED**
Beecham House Great West Road
Brentford, Middlesex(GB)

(72) Inventor: **Walton, Jill Mary**
15 Maple Lodge
Whitefield Close Putney, SW15(GB)

(74) Representative: **Russell, Brian John et al,**
European Patent Attorney Beecham Pharmaceuticals
Yew Tree Bottom Road
Epsom Surrey, KT18 5XQ(GB)

(54) **Calcium enhanced interferon production process.**

(57) Interferon may be produced from human fibroblasts by induction with a natural double-stranded RNA or a derivative thereof, using 10 to 25 m$M$ calcium ions to enhance the yield. The yield may further be increased using DEAE-Dextran.

EP 0 030 094 A1

INTERFERON PRODUCTION

This invention relates to an improved method for the production of human interferon.

Interferon is the name given to glycoprotein which is produced by some cells in response to stimuli such as viral infection and interferon inducing agents such as natural or synthetic ribopolynucleotides. The increasing importance of interferon as a therapeutic agent in the management of viral infections such as Herpes types I and II and malignant diseases such as osteosarcoma has focused much attention on the problem of producing large quantities of interferon from cell cultures.

The production of interferon in cell culture from normal cells with the aid of a simple inducer appears to be unsatisfactory for large scale production. One solution to this problem was to use high yielding abnormal cells, since it was found that transformed and tumor cell lines often produce high interferon yields.

However, while interferon so produced may be satisfactory for non-clinical purposes it is doubtful whether material produced in this way would be acceptable for medicinal purposes.

A second solution is to try to enhance interferon yields from interferon producing cells. To date many different and indeed often conflicting methods have been suggested in the literature for improving interferon production from normal and abnormal cells. For example it has been suggested [Y Tan et al, 1970, Proc Natl Acad Sci USA, 67, 464; J Vilcek et al, 1971, J Virol, 7, 588, and Y Tan et al, 1977, J Gen Virol, 34, 401] that the use of inhibitors of protein and RNA synthesis such as cycloheximide combined with actinomycin D is required to enhance production of interferon from some cell lines following induction with the synthetic polynucleotide polyinosinic: polycytidylic acid (poly IC). It has also been suggested that optimal results are obtained from other cell cultures by pretreating with interferon prior to induction with poly IC. In addition it has been reported [A Meager et al, 1978, FEBS Letters, 87,303] that it is possible to improve interferon yields following induction with poly IC by treating certain human cell lines with 12 mM calcium chloride solution continuously for a period commencing 12 hours prior to induction and continuing for a period of 20 hours after induction when interferon is harvested. However, it is to be noted that the best interferon yields obtained by this method were given by a transformed cell line and osteosarcoma (tumor) cells, that is to say cells which do not possess the properties of normal human cells.

It is recognised that while specific combinations of inducer and enhancer may be found to produce improved and high levels of interferon, the same enhancer may be completely unable to produce satisfactory levels of interferon with another inducer. Moreover to date no discernable rule emerges from the prior art which allows one to predict with confidence how improvements to interferon yields may be obtained.

It has now been discovered that particularly advantageous levels of interferon may be obtained when a natural double stranded ribonucleic acid is used in the presence of dissolved calcium to induce interferon from interferon producing human cells.

Accordingly the invention provides a process for producing human interferon in vitro, which method comprises contacting interferon producing human cells with an interferon inducing medium comprising:

(i) a non-toxic quantity of either (a) a double-stranded ribonucleic acid of natural origin or (b) a double-stranded derivative of a double-stranded ribonucleic acid of natural origin; and (ii) from 10 to 25 mM dissolved calcium ions, under physiological condition of temperature and pH, and thereafter separating the interferon so formed.

The term "double-stranded" used in connection with ribonucleic acid refers to the characteristic whereby two ribonucleic acid molecules are associated by hydrogen bonding between complementary bases in each molecule. Ribonucleic acids may vary in the degree of "double-strandedness".

The term "double-stranded ribonucleic acid of natural origin" means any double-stranded ribonucleic acid which is isolatable from a naturally-occurring source (eg those sources listed earlier in this specification), and excludes synthetic double-stranded ribonucleic acids such as Poly I : Poly C, Poly A : Poly U and Poly G : Poly C.

The term "double-stranded derivative of a double-stranded ribonucleic acid of natural origin" means any double-stranded ribonucleic acid of natural origin which has been subjected to a chemical or biochemical (eg enzymatic) reaction which alters the primary and/or secondary and/or tertiary structure (eg the N-oxides described in our British Patent No 1 284 150 or the alkali-modified double-stranded ribonucleic acids in our British Patent No 1 356 263 provided that the resultant derivative retains a substantial degree of base-pairing between complementary strands.

The double-strandedness of a double-stranded ribonucleic acid or a derivative of a double-stranded ribonucleic acid can be measured by two parameters known as the hyperchromicity and Tm. These parameters are obtained by recording the ultra violet absorption of the material at 258 mμ while gradually raising the temperature of the material. The u.v. absorption value of a double-stranded material at this frequency increases with increasing temperature until a constant value is reached, corresponding to the absorption of the thermally denatured (ie single-stranded) ribonucleic acid. The difference between the two extremes of absorption expressed as a percentage of the absorption of the double-stranded material is termed the "hyperchromicity" of that material.

When the u.v. absorption at 258 mμ of a double-stranded material is plotted against temperature, it is found that the absorption is greater than at low temperatures. The temperatures at which the absorption is mid-way between the absorption of the double-stranded material and that of the thermally denatured (ie single-stranded) material is called the Tm of the material.

The term "natural ds RNA" in this specification means either double-stranded ribonucleic acid of natural origin or a double-stranded derivative thereof.

Preferred sources of double-stranded ribonucleic acid include the virus like particles found in certain of the Penicillia, eg P chrysogenum (British Patent No 1,170,929), P stoloniferum (Banks et al, Nature 218, 542 (1968)), P cyaneofulyum (Banks et al, Nature 223, 155 (1968)), and in certain of the Aspergilli eg, A niger and A foetidus (our co-pending Patent Application No 13826/70). Preferably also the component (a) or (b) should be capable of inducing interferon production in live mammals. (This can be confirmed by the method of Lampson et al. G P Lampson, A A Tytell, A K Field, N M Nemes and Mr Hillerman Proc Nat Acad Sci, 58 (1967), 782).

Additionally we have found that in vitro interferon production is further enhanced when induction is carried out in the presence of dissolved calcium and diethylaminoethyl dextran [DEAE - Dextran].

Accordingly in a further aspect the invention provides a method for producing human interferon, which method comprises contacting interferon producing human cells with an interferon inducing medium comprising a non-toxic quantity of natural ds RNA, not more than 1000 mg per litre of DEAE-Dextran and not less than 10 but not more than 25 millimoles of dissolved calcium per litre, under physiological conditions of temperature and pH, and thereafter isolating the interferon so formed.

In order to put this invention into practice it is generally most convenient to culture an appropriate quantity of cells by any of the standard techniques, and thereafter to treat the cell population with the inducer in the presence of dissolved calcium and optionally DEAE-Dextran.

The method of this invention may be put into effect with either normal or transformed cells, but it is preferrred to use normal cells since they produce clinical grade material.

The method of this invention may be applied to cells which grow in monolayers.

The monolayer culture technique requires that the cell is anchored to some mechanical support, such as a plate or bead, and supplied with a nutrient medium    by submersion or perfusion while being mantained at a physiological pH and temperature.  Methods for culturing cells in monolayer are also described by J Paul, loc cit. Alternative methods for culturing sheet forming cells have been decribed by A L van Wezel et al, Process Biochemistry, March 1978, 6-28; W Wohler et al, J Exptl Cell Res, 74, 1972, 571; Spier, Biotech Bioeng, 18, 649-689, 1976 and Belgian Patent No 842,002.

Examples of suitable sheet forming cells include epithelial cells and fibroblasts.  It is also preferred to put the method of the invention into practice using sheet forming cells, in particular diploid fibroblasts.  MRC-5 diploid fibroblasts are preferred.

Examples of suitable nutrient media include Eagle's, Fischer's, Ham's, Leibovitz', McCoy's, Neumann and Tytell's, Puck's, Swim's, Trowell's or Waymouth's medium, also 199, NCTC 109, NCTC 135, CMRL 1066, or RPML medium.

When a confluent cell sheet is obtained, the cells are contacted with natural ds RNA in the presence of dissolved calcium.  This may be done by draining off the nutrient medium used for culturing the cells and replacing it by an inducing medium which comprises a minimum essential medium containing the appropriate levels of dissolved calcium, natural ds RNA and preferably also DEAE - Dextran.

It should be noted that dissolved calcium ions are not required in the harvest medium to obtain the enhanced yield of interferon. Indeed, it is preferred that no additional calcium ions are included in the harvest medium in order to simplify separation of the interferon.

Any water soluble non-toxic calcium salt may be used in order to achieve the required concentration of dissolved calcium. For convenience, the chloride is preferred. Sufficient calcium salt must be added such that the dissolved calcium concentration is not less than 10 mmolar and not more than 25 mmolar, a concentration from 12 to 20 mmolar being more advantageous and a concentration from 15 to 20 mmolar being most apt.

The quantity of natural ds RNA is not particularly critical to this invention, and appropriate quantities may be determined by trial and error. However, in general low concentrations, ie below 100 $\mu gL^{-1}$, produce inconveniently low quantities of interferon, and at higher concentrations for example above 100 $mgL^{-1}$ some toxic signs are observed in the cell culture. Of course the precise concentration at which natural ds RNA displays toxicity varies from cell line to cell line, but in general levels of natural ds RNS which produce substantial toxic effects should not be employed. Suitable concentrations of natural ds RNA lie in the range 0.1 to 50 $mgL^{-1}$ inclusive, a concentration of 1 to 10 $mgL^{-1}$ being most convenient.

DEAE-Dextran is a polymer of DEAE-glucose of molecular weight between $10^3$ to $10^8$. DEAE-Dextran more suitable for use in the method of this invention generally has a molecular weight of $2 \times 10^4$ to $2 \times 10^6$. A number of DEAE-Dextrans of different molecular weight are available commercially, or may be made by known methods. DEAE-Dextran molecular weight $5 \times 10^5$ as supplied by Pharmacia is particularly convenient. The concentration of DEAE-Dextran included in the induction medium will not exceed 1000 $mgL^{-1}$, 100 $mgL^{-1}$ being particularly convenient.

The time for which the induction should be carried out will of course vary with the type of cell line employed, the concentration of inducer, dissolved calcium and DEAE-Dextran. The optimum time for any particular set of circumstances may be conveniently determined by trial and error, that is to say by titrating the interferon harvested after a series of given time periods.

The induction step is carried out under physiological pH conditions ie pH 6.5 to 8.0, and at a temperature in the range $2^{\circ}-40^{\circ}C$, $34^{\circ}-38^{\circ}$ being most apt.

When the induction step is completed, the inducing medium is drained, optionally washed by contacting with a suitable nutrient medium, and thereafter contacted with a harvest medium. The harvest medium is left in contact with the cells until interferon production has ceased. Suitable harvest media include the nutrient media previously discussed. The length of time for which cells are left in harvest medium depends on the length of time for which the cell continues to produce interferon. This will, of course, vary depending upon the type of cell which is employed, but may be determined by titrating aliquots at given time intervals.

When all interferon production has ceased, the harvest medium is drained from the cells, and the interferon recovered by standard techniques.


The following Example illustrates this invention.

Example

MRC-5 cells were grown to confluency in 1.5 cm wells of Linbro tissue culture plates using 1.0 ml minimum essential (Eagle's) medium containing 8% foetal calf serum, 1% penicillin-streptomycin solution (Flow Laboratories) and 2.2 $gL^{-1}$ sodium bicarbonate.

After removal of growth medium the cultures were washed with phosphate buffered saline and incubated in 1.0 ml induction medium for 2 hours. Induction medium consists of minimum essential (Eagle's) medium containing 1% penicillin-streptomycin solution, 2.2 $gL^{-1}$ sodium bicarbonate and 10 $mgL^{-1}$ BRL 5907*. DEAE-Dextran (Pharmacia; 5 x $10^5$ mol wt), where present, was used at 100 $mgL^{-1}$. $CaCl_2$ was added from a 1 $molL^{-1}$ sterile solution to give a final concentration in the induction medium of 10-20 $mmolL^{-1}$.

At the end of the induction period, the induction medium was removed and replaced with 0.5 ml collection medium. Cultures were incubated for a further 20 hours. Collection medium consists of minimum essential (Eagle's) medium, 1% foetal calf serum, 1% non-essential amino acids, 1% penicillin-streptomycin solution and 2.2 $gL^{-1}$ sodium bicarbonate. At the end of this second incubation period, the interferon-containing collection medium was removed from the cultures and stored at $-40^{\circ}C$, until assay for human interferon by a modification of the assay for chick interferon reported by Viehhauser (Viehhauser, G, (1977) Applied and Environ Microbiol, 33, 740). The amounts of interferon produced in the presence and absence of $CaCl_2$ are shown in Table 1.

---

* BRL 5907 is a double-stranded RNA isolated from virally infected strains of Penicillin chrysogenum.

## Assay Method

$V_3$ monkey cells are grown to confluency in conventional roux bottles, and a cell suspension prepared by trypsinisation. The cell suspension is infected with Semliki Forest Virus, and aliquots of the suspension are dispensed into dilutions of the interferon samples (unknowns and standards) in 96-well microtitre plates. The plates are incubated for 3 days at $37^{\circ}C$, before fixing the cells and staining with carbol fuchsin. The end point titre is the dilution of sample which results in the cells staining to 50% of the uninfected cell control cultures. The concentration of interferon in reference i.u./ml is calculated by comparison to the performance of a laboratory standard in the same assay. The laboratory standard is calibrated, in turn, against an NIH International standard.

- 12 -

Table 1  **

Experiment 1

| Induction regime | Interferon yield (i.u./ ml collection medium) |
|---|---|
| $10 \text{ mgL}^{-1}$ 5907 | <88 * |
| $10 \text{ mgL}^{-1}$ 5907 + 10 mM $CaCl_2$ | 1,354 |
| $10 \text{ mgL}^{-1}$ 5907 + 25 mM $CaCl_2$ | 2,100 |
| $10 \text{ mgL}^{-1}$ 5907 + 25 mM $CaCl_2$ + 100 $\text{mgL}^{-1}$ DEAE-D | 3,570 |

Experiment 2

| Induction regime | Interferon yield (i.u./ ml collection medium) |
|---|---|
| $10 \text{ mgL}^{-1}$ 5907 + 100 $\text{mgL}^{-1}$ DEAE-D | 1,542 |
| $10 \text{ mgL}^{-1}$ 5907 + 100 $\text{mgL}^{-1}$ DEAE-D + 10 mM $CaCl_2$ | 5,400 |
| $10 \text{ mgL}^{-1}$ 5907 + 100 $\text{mgL}^{-1}$ DEAE-D + 20 mM $CaCl_2$ | 7,700 |

* 88 i.u./ml minimum detectable level of interferon in the assay.

** 5907 means double-stranded RNA isolated from virally infected strains of Penicillium chrysogenum.
DEAE-D means DEAE-Dextran.

- 13 -

CLAIMS

1. A process for producing human interferon *in vitro* characterised by contacting interferon producing human cells with an interferon inducing medium comprising:

    (i)   a non-toxic quantity of either (a) a double-stranded ribonucleic acid of natural origin; or (b) a double-stranded derivative of a double-stranded ribonucleic acid of natural origin; and

    (ii)  from 10 to 25 m$\underline{M}$ dissolved calcium ions,

    under physiological conditions of temperature and pH, and thereafter separating the interferon so formed.

2. A process as claimed in claim 1 wherein the quantity of natural double-stranded RNA or a double-stranded derivative of natural double-stranded RNA in the inducing medium is in the range 100 $\mu gL^{-1}$ to 100 $mgL^{-1}$.

3. A process as claimed in claim 2 wherein the quantity of natural double-stranded RNA or a double-stranded derivative of natural double-stranded RNA in the inducing medium is in the range 0.1 to 50 $mgL^{-1}$.

4. A process as claimed in any one of claims 1 to 3 wherein the quantity of dissolved calcium ions in the inducing medium is in the range from 12 to 20 m$\underline{M}$.

5. A process as claimed in claim 4 wherein the quantity of dissolved calcium ions in the inducing medium is in the range from 15 to 20 m$\underline{M}$.

6. A process as claimed in any one of claims 1 to 5 wherein the dissolved calcium ions are provided by a solution of calcium chloride.

7. A process as claimed in any one of claims 1 to 6 wherein the inducing medium further comprises up to 1000 $mgL^{-1}$ of DEAE-Dextran.

8. A process as claimed in claim 7 wherein the inducing medium comprises about 100 $mgL^{-1}$ of DEAE-Dextran.

9. A process as claimed in any one of claims 1 to 8 wherein the interferon producing human cells are normal diploid fibroblasts.

10. A process as claimed in any one of claims 1 to 8 wherein human MRC-5 fibroblast cells are cultured in a nutrient medium to provide a confluent cell sheet, the nutrient medium is removed and the cells are contacted with inducing medium comprising a minimum essential medium containing 10 to 20 m$\underline{M}$ calcium chloride, 1-10 $mgL^{-1}$ BRL 5907 and 100 $mgL^{-1}$ of DEAE-Dextran of $5 \times 10^{5}$ molecular weight, the inducing medium is removed and the cells are cultured in minimum essential medium containing added serum, and thereafter separating the interferon, so formed.

# EUROPEAN SEARCH REPORT

**European Patent Office**

Application number

EP 80 30 4065

## DOCUMENTS CONSIDERED TO BE RELEVANT

| Category | Citation of document with indication, where appropriate, of relevant passages | Relevant to claim |
|---|---|---|
| D,X | CHEMICAL ABSTRACTS, vol. 88, no. 21, 22nd May 1978, page 417, no. 150305j Columbus, Ohio, U.S.A. A. MEAGER et al.: "Stimulation of interferon yields from cultured human cells by calcium salts" & FEBS LETT. 1978, 87(2), 303-307 * Abstract * | 1-10 |
| X | CHEMICAL ABSTRACTS, vol. 89, no. 19, 6th November 1978, page 410, no. 161415s Columbus, Ohio, U.S.A. B.W. BOOTH et al.: "Increase by calcium in production of interferon by L929 cells induced with polyriboinosinate-polyribocytidylate complex" & J. GEN. VIROL. 1978, 40(2), 485-488 * Abstract * | 1-10 |
| | CHEMICAL ABSTRACTS, vol. 91, no. 5, 30th July 1979, page 310, no. 35624n Columbus, Ohio, U.S.A. T.V. MAMONTOVA et al.: "Effect of calcium ions on the efficiency of translation by bacteria of interferon mRNA" & ACTA VIROL. (ENGL. ED.) 1979, 23(3), 271 * Abstract * | 1-10 |
| | US - A - 3 679 654 (R. MAES) ./. | |

### CLASSIFICATION OF THE APPLICATION (Int. Cl.3)

C 12 P 21/00//
C 12 R 1/91

### TECHNICAL FIELDS SEARCHED (Int. Cl.3)

A 61 K 31/70
45/02
C 12 P 21/00
1/00
C 12 R 1/91

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

The present search report has been drawn up for all claims

| Place of search | Date of completion of the search | Examiner |
|---|---|---|
| The Hague | 19-01-1981 | GALLIGANI |

EPO Form 1503.1 06.78

0030094

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| | * Page 1, left-hand column, lines 24-30; claims 1,2; page 2, right-hand column, lines 11-24 * | | |
| | -- | | |
| D,A | GB - A - 1 525 022 (BEECHAM) <br> * Page 5, lines 24-74; claims 13-16 * | 1-10 | |
| | -- | | |
| P,A | EP - A - 0 014 050 (BEECHAM) <br> * Whole document * | 1-10 | TECHNICAL FIELDS SEARCHED (Int. Cl. ³) |
| | ---- | | |

EPO Form 1503.2  06.78